# EUROPEAN PATENT APPLICATION

(11) **EP 4 015 539 A1**
(43) Date of publication of application: **22.06.2022**
(21) Application number: 20425057.5
(22) Date of filing: 16.12.2020
(51) Int. Cl.: C07K 16/42, A61P 35/00, A61P 35/02, C07K 16/06

(54) **METHOD FOR PRODUCING HUMAN MONOCLONAL ANTI-IDIOTYPE ANTIBODIES**

(71) Applicant: Cryolab S.r.l., 00133 Roma (IT)
(72) Inventor: DANIELE, Nicola, 00128 Roma (IT); AGOSTINI, Francesca, 00134 Roma (IT)
(74) Representative: Ferreccio, Rinaldo

(57) **Abstract**

A method for producing human monoclonal anti-idiotype antibodies against the variable region of the antibody which is an integrated part of the B-cell receptor (BCR) of a patient suffering from a clonal B-cell lymphoproliferative disorder, wherein the method comprises the following steps: a) purifying IgM antibodies produced by B cells obtained from a blood sample from the patient suffering from a B-cell lymphoproliferative disorder; b) purifying B cells from blood samples obtained from one or more healthy individuals; c) contacting the IgM antibodies obtained from step a) with the B cells obtained from step b), thus forming IgM antibody-B cell complexes; d) purifying and dissociating the complexes formed in step c) thus obtaining B cells producing said human monoclonal anti-idiotype antibodies.

SEQUENCE LISTING

<110> Cryolab S.r.l.

<120> Method for producing human monoclonal anti-idiotype antibodies

<130> CLB001BEP

<160> 35

<170> BiSSAP 1.3.6

<210> 1
<211> 21
<212> DNA
<213> Unknown

<220>
<223> none

<400> 1
gggaattctc acaggagacg a                                                21

<210> 2
<211> 21
<212> DNA
<213> Unknown

<220>
<223> none

<400> 2
tcaccatgga ctgcacctgg a                                                21

<210> 3
<211> 22
<212> DNA
<213> Unknown

<220>
<223> none

<400> 3
ccatggacac actttgctcc ac                                               22

<210> 4
<211> 23
<212> DNA
<213> Unknown

<220>
<223> none

<400> 4
tcaccatgga gtttgggctg agc                                              23

<210> 5
<211> 25
<212> DNA
<213> Unknown

<220>
<223> none

<400> 5
agaacatgaa acacctgtgg ttctt                                             25

<210> 6
<211> 20
<212> DNA
<213> Unknown

<220>
<223> none

<400> 6
atggggtcaa ccgccatcct                                                   20

<210> 7
<211> 23
<212> DNA
<213> Unknown

<220>
<223> none

<400> 7
acaatgtctg tctccttcct cat                                               23

<210> 8
<211> 20
<212> DNA
<213> Unknown

<220>
<223> none

<400> 8
caggagacga gggggaaaag                                                   20

<210> 9
<211> 20
<212> DNA
<213> Unknown

<220>
<223> none

<400> 9

caggtgcagc tggtgcagtc                                                    20

<210> 10
<211> 20
<212> DNA
<213> Unknown

<220>
<223> none

<400> 10
cagatcacct tgaaggagtc                                                    20

<210> 11
<211> 20
<212> DNA
<213> Unknown

<220>
<223> none

<400> 11
gaggtgcagc tggtggagtc                                                    20

<210> 12
<211> 20
<212> DNA
<213> Unknown

<220>
<223> none

<400> 12
caggtgcagc tgcaggagtc                                                    20

<210> 13
<211> 20
<212> DNA
<213> Unknown

<220>
<223> none

<400> 13
gaggtgcagc tggtgcagtc                                                    20

<210> 14
<211> 20
<212> DNA
<213> Unknown

<220>
<223> none

<400> 14
caggtacagc tgcagcagtc                                                    20


<210> 15
<211> 30
<212> DNA
<213> Unknown


<220>
<223> none

<400> 15
aggtctagag aaaagggttg gggcggatgc                                         30


<210> 16
<211> 18
<212> DNA
<213> Unknown


<220>
<223> none

<400> 16
aacagaggca gttccaga                                                      18


<210> 17
<211> 18
<212> DNA
<213> Unknown


<220>
<223> none

<400> 17
tgtggccttg ttggcttg                                                      18


<210> 18
<211> 21
<212> DNA
<213> Unknown


<220>
<223> none

<400> 18
gctcagctcc tggggctcct g                                                  21


<210> 19
<211> 21
<212> DNA
<213> Unknown

<220>
<223> none

<400> 19
ctggggctgc taatgctctg g                                                21


<210> 20
<211> 21
<212> DNA
<213> Unknown


<220>
<223> none

<400> 20
ttcctcctgc tactctggct c                                                21


<210> 21
<211> 21
<212> DNA
<213> Unknown


<220>
<223> none

<400> 21
cagacccagg tcttcatttc t                                                21


<210> 22
<211> 20
<212> DNA
<213> Unknown


<220>
<223> none

<400> 22
cgacatccag atgacccagt                                                  20


<210> 23
<211> 19
<212> DNA
<213> Unknown


<220>
<223> none

<400> 23
cgatattgtg atgacccag                                                   19


<210> 24
<211> 22
<212> DNA
<213> Unknown

<220>
<223> none

<400> 24
cgaaattgtg ttgacgcagt ct                                                22


<210> 25
<211> 20
<212> DNA
<213> Unknown


<220>
<223> none

<400> 25
cgacatcgtg atgacccagt                                                   20


<210> 26
<211> 20
<212> DNA
<213> Unknown


<220>
<223> none

<400> 26
cctctcctcc tcaccctcct                                                   20


<210> 27
<211> 18
<212> DNA
<213> Unknown


<220>
<223> none

<400> 27
ctcctcactc agggcaca                                                     18


<210> 28
<211> 19
<212> DNA
<213> Unknown


<220>
<223> none

<400> 28
atggcctgga tccctctcc                                                    19


<210> 29
<211> 30

<212> DNA
<213> Unknown

<220>
<223> none

<400> 29
tattagatct ccagtctgtg ctgactcagc                                        30

<210> 30
<211> 30
<212> DNA
<213> Unknown

<220>
<223> none

<400> 30
tattagatct ccagtctgcc ctgactcagc                                        30

<210> 31
<211> 30
<212> DNA
<213> Unknown

<220>
<223> none

<400> 31
caccagatct ctcctatgag ctgactcagc                                        30

<210> 32
<211> 24
<212> DNA
<213> Unknown

<220>
<223> none

<400> 32
tttcaactgc tcatcagatg gcgg                                              24

<210> 33
<211> 20
<212> DNA
<213> Unknown

<220>
<223> none

<400> 33
aagatgaaga cagatggtgc                                                   20

<210> 34
<211> 20
<212> DNA
<213> Unknown

<220>
<223> none

<400> 34
agctcctcag aggagggcgg 20

<210> 35
<211> 26
<212> DNA
<213> Unknown

<220>
<223> none

<400> 35
cattccatgg gggaacagag tgaccg 26

## Description

### FIELD OF THE INVENTION

The present invention generally relates to the field of pharmaceutical industry and biotechnology; in particular, the invention relates to a method for producing human monoclonal anti-idiotype antibodies.

### BACKGROUND

B-cell lymphoproliferative disorders are diseases which involve uncontrolled lymphocyte growth. These diseases include tumors such as multiple myeloma, Hodgkin lymphoma and chronic lymphocytic leukemia (CLL) and other conditions such as monoclonal B-cell lymphocytosis.

Lymphoproliferative disorders may originate both in lymphatic tissues (as in the case of lymphoma) and in the bone marrow (as in the case of leukemia and myeloma). The course and treatment of the disease broadly vary depending on the type of the cancer and other individual factors.

In the USA, 71,850 new cases of non-Hodgkin lymphomas with 19,790 deaths (corresponding to 3.4% of all deaths due to tumors) are recorded every year. The prevalence (i.e. the number of patients suffering from the disease) is about 550,000 patients, corresponding to 17.19 cases every 10,000 inhabitants, with an average survival of 70% 5 years after diagnosis. Non-Hodgkin lymphoma therapy is still today an important challenge for oncologists.

B-cell non-Hodgkin lymphomas account for 85% of the total (the remaining 15% originate from T lymphocytes).

The known B-cell lymphoma treatment methods consist of chemotherapy cycles for diffuse tumors and radiotherapy cycles for the localized ones.

Any patients who do not respond to the above treatment methods or show relapses undergo immunotherapy and radio-immunotherapy, which are based on the use of monoclonal antibodies against an antigen present on mature B lymphocytes, named CD20.

In particular, in immunotherapy, the anti-CD20 antibody is used alone, while in radio-immunotherapy it is coupled with a radioisotope.

It is also known that patients resistant to or relapsing to immunotherapy or radio-immunotherapy may be treated by CAR-T ("Chimeric Antigen Receptor T cell therapies") or by marrow transplant (autologous, allogeneic or from stem cells taken from umbilical cord).

It is known that CAR-Ts indifferently destroy both neoplastic and healthy B lymphocytes. Moreover, both CAR-Ts and marrow transplants are very expensive and, particularly the latter, have high morbidity and mortality; in fact, severe infections and second neoplasms caused by the suppression of the immune system due to chemotherapy, pre-surgery radiotherapy and/or graft-versus-host-disease may occur.

Chronic lymphocytic leukemia (CLL) is a lymphoproliferative pathology, i.e. a cancer of the lymphatic system, characterized by an accumulation of B lymphocytes in the peripheral blood, in the bone marrow and in lymphatic organs (lymph nodes and spleen); its diagnosis is based on complete blood count, medullar biopsy (possibly combined with bone biopsy) and cytofluorometry. Regarding B-cell chronic lymphocytic leukemia, several subgroups having different prognosis depending on the expression of specific membrane antigens based on cytofluorometry can be identified.

Both the onset and the clinical course are very variable considering different cases. A quarter of patients do not show any symptom or clinical sign, and the diagnosis is made by chance after performing the ordinary laboratory tests. But in a limited number of cases the disease occurs with anemia, thrombocytopenia or, more rarely, with a context of autoimmune hemolytic anemia. Typically, the disease begins with lymphadenomegalia which extensively involves the main lymph nodal sites, often accompanied by hepato-splenomegalia.

The most common treatment of B-cell chronic lymphocytic leukemia involves administration of fludarabine and cyclophosphamide; in patients with limited and painless disease, especially if elderly people, it is common practice not to perform any treatment until a clear progression of the disease.

In recent years, the so-called "target therapy", whose purpose is to counteract the specific mechanisms of the carcinogenesis process of single tumors, has considerably emerged, for example by ibrutinib, idelalisib and venetoclax administration.

However, about a third of patients do not respond to the treatments and, in this case too, the only possible treatments are CAR-T therapies and marrow transplant, which involving the above disadvantages.

At the beginning of the 1990s, Ronald Levi observed that B-cell non-Hodgkin lymphomas originate from the neoplastic transformation of a single lymphocyte. For this reason, all tumor cells constituting it are characterized by the presence on the cell membrane of the same BCR (B-cell receptor) which uniquely characterizes tumor cells compared to healthy cells (T.A. Davis, D.G. Maloney, D..K Czerwinski, T.M. Liles, R Levi. "Anti-idiotype antibodies can induce long-term complete remissions in non-Hodgkin's lymphoma without eradicating the malignant clone" Blood 1998 Aug 15;92(4):1184-90). Therefore, the BCR on the surface of neoplastic cells of non-Hodgkin lymphomas and chronic lymphocytic leukemia is an ideal target for the development of highly specific therapies.

It is known that the B-cell receptor or BCR is a transmembrane receptor protein located on the external surface of B cells, and is composed of two fractions: the ligand-binding fraction, consisting of a transmembrane antibody of a single isotype (IgD, IgM, IgA or IgE), and the signal transduction fraction, consisting of a heterodimer called Ig-α/Ig-β (CD79).

Levi developed a technique for producing monoclonal antibodies against the variable region of the BCR antibody present on the membrane of tumor B cells. Since the variable region of an antibody is referred to as "idiotype", the antibodies produced against the antibody present on the surface of tumor cells were referred to as "anti-idiotype antibodies".

Levi's method allowed to make anti-idiotype antibodies that specifically recognize the BCR idiotype which uniquely characterizes the neoplastic cells in patients with non-Hodgkin lymphomas, thereby allowing to maximize treatment efficacy and minimizing the risk of side effects.

However, the monoclonal antibodies produced by Levi were murine and, for this reason, they were recognized as foreign by the patient's immune system, thereby inducing after a period of time the production of neutralizing antibodies that jeopardized their efficacy. Moreover, the quantity of antibody produced was limited (no more than 15 g per patient), the production times were very long (about six months) and the costs were unaffordable, so that it was impossible to apply this promising methodology from research to clinical practice.

Also the technique currently used for producing monoclonal antibodies leads to the production of murine monoclonal antibodies, and it is based on the method developed in 1975 by Milstein e Kohler (Nature, 256, 495 (1975)) and later used by Levi for the above-mentioned production of murine monoclonal anti-idiotype antibodies.

In brief, the general production scheme of murine monoclonal antibodies first comprises the immunization of a mouse with the antigen against which the antibodies have to be produced. B lymphocytes extracted from the mouse's spleen are then "fused" with plasma cells having lost their ability of producing antibodies. In this way, hybridomas that are able to produce large amounts of the murine monoclonal antibody of interest are obtained.

It is clear that the above known method involves a certain complexity, since it includes the immunization of laboratory animals, their sacrifice and the treatment of the spleen to obtain B lymphocytes. Moreover, as previously mentioned, this method provides murine antibodies, which entails the disadvantages described above about their tolerability in a human patient.

It is also known that, to carry out the whole range of biological activities, the Fc fragment of an antibody binds to the Fc receptor on the surface of macrophages, neutrophilic granulocytes and NK lymphocytes, inducing the antibody-dependent cellular cytotoxicity (ADCC) phenomenon and the complement activation following opsonization. However, said activity is species-specific, therefore only human antibodies are able to interact with the human immune system cells.

In the light of the above, the technical problem underlying the present invention is to provide a method for producing human monoclonal anti-idiotype antibodies against the BCRs on the surface of lymphocytes of patients suffering from B-cell lymphoproliferative disorders, said method being rapid, inexpensive and substantially free of the disadvantages of the known methods.

### SUMMARY OF THE INVENTION

The above-mentioned technical problem was solved by the present invention by providing a method for producing human monoclonal anti-idiotype antibodies against the variable region of the antibody comprised in the B-cell receptor (BCR) of a patient suffering from a B-cell lymphoproliferative disorder, said method comprising the following steps:
a) purifying IgM antibodies produced by B cells obtained from a blood sample from said patient suffering from a B-cell lymphoproliferative disorder;
b) purifying B cells from blood samples obtained from one or more healthy individuals;
c) contacting the above-mentioned IgM antibodies obtained from step a) with the above-mentioned B cells obtained from step b), thus forming IgM antibody-B cell complexes;
d) purifying and dissociating the complexes formed in step c) thus obtaining B cells producing the above-mentioned human monoclonal anti-idiotype antibodies.

Preferably, step a) of purifying IgM antibodies comprises the following steps:
i) centrifuging in density gradient a blood sample from a patient suffering from a B-cell lymphoproliferative disorder, thus obtaining and isolating peripheral mononuclear cells (PBMC);
ii) contacting the above-mentioned peripheral mononuclear cells (PBMC) with monoclonal anti-B-cell antibodies linked to magnetic beads and performing an immunomagnetic separation, thus obtaining purified B cells;
iii) culturing the purified B cells obtained from step ii);
iv) purifying the IgM antibodies produced by said B cells in culture.

In another equally preferred embodiment of the present invention, step a) of purifying IgM antibodies comprises the following steps:
i) contacting a blood sample from a patient suffering from a B-cell lymphoproliferative disorder with monoclonal anti-B-cell antibodies linked to magnetic beads and performing an immunomagnetic separation, thus obtaining purified B cells;
ii) culturing the purified B cells obtained from step i) ;
iii) purifying the IgM antibodies produced by said B cells in culture.

Preferably, step b) of purifying B cells comprises the following steps:
i) centrifuging in density gradient a blood sample from a healthy individual, thus obtaining and isolating peripheral mononuclear cells (PBMC);
ii) contacting said peripheral mononuclear cells (PBMC) with monoclonal anti-B-cell antibodies linked to magnetic beads and performing an immunomagnetic separation, thus obtaining purified B cells.

In another equally preferred embodiment of the present invention, step b) comprises contacting a blood sample from a healthy individual with monoclonal anti-B-cell antibodies linked to magnetic beads and performing an immunomagnetic separation, thus obtaining purified B cells.

The above-mentioned monoclonal anti-B-cell antibodies linked to magnetic beads are typically monoclonal anti-CD 19 antibodies or monoclonal anti-CD20 antibodies.

Preferably, the above-mentioned centrifugation in density gradient is performed in an aqueous solution having a density of 1.077 g/ml at 20°C and comprising a polymer of polysucrose and sodium diatrizoate having a molecular weight between 350.000 and 450.000 Da.

In an embodiment of the present invention, the above-mentioned blood sample is a buffy coat of one or more healthy individuals.

Preferably, the above-mentioned step c) of forming IgM antibody-B cell complexes comprises the following steps:
i) biotinylating the IgM antibodies obtained from step a);
ii) contacting the B cells obtained from step b) with human polyclonal IgMs under conditions such as to saturate the Fc receptors of said B cells;
iii) contacting the biotinylated IgM antibodies obtained from step i) with the B cells obtained from step ii), thus obtaining B cell-biotinylated IgM complexes;
iv) contacting said B cell-biotinylated IgM complexes with magnetic beads associated to streptavidin, avidin or neutravidin, thus obtaining B cell-IgM-magnetic bead complexes;
v) isolating said complexes obtained from step iv) by magnetic separation;
vi) isolating said IgM antibody-B cell complexes by dissociating the complexes obtained from step v) from the magnetic beads.

Preferably, the above-mentioned B-cell lymphoproliferative disorder is selected from the group comprising follicular lymphoma (FL), chronic lymphocytic leukemia (CLL), B-cell non-Hodgkin lymphoma, acute lymphoblastic leukemia, hairy cell leukemia (HCL), B-cell lymphomas including diffuse large B-cell lymphoma (DLBCL), multiple myeloma, Waldenström's macroglobulinemia (WM), post-transplant lymphoproliferative disorder (PTLD) and Epstein-Barr virus-associated B-cell lymphoproliferative disorders.

Preferably, the B-cell lymphoproliferative disorder is chronic lymphocytic leukemia (CLL) or B-cell non-Hodgkin lymphoma.

In another aspect thereof, the present invention also relates to a human monoclonal anti-idiotype antibody obtained according to the above-described method of the present invention.

By "anti-idiotype antibody", as used herein, is meant an antibody which specifically binds to the variable region of another antibody; therefore, , the anti-idiotype antibody specifically binds to another antibody. An idiotype is the antigenic determinant characteristic of the variable portion of an antibody.

As used herein, the terms "antibody" and "immunoglobulin" are used interchangeably.

Native antibodies and immunoglobulins are usually heterodimeric glycoproteins of about 150,000 Dalton, composed of two identical light chains (L) and two identical heavy (H) chains. Each light chain is linked to the heavy chain by one covalent disulfide bond, while the number of disulfide bonds varies between the heavy chains of different immunoglobulin isotypes.

Each heavy and light chain has regularly spaced intrachain disulfide bonds. Each heavy chain has at one end a variable domain (VH) followed by a number of constant domains; each light chain has a variable domain at one end (VL) and a constant domain at its other end.

In a correctly folded antibody, the constant domain of the light chain is aligned with the first constant domain of the heavy chain, and the light chain variable domain is aligned with the variable domain of the heavy chain.

Human immunoglobulin classes are defined on the basis of their heavy chain constant domain composition, and are named IgG, IgM, IgA, IgE, and IgD. In humans, the IgG-class and IgA-class antibodies are further divided into subclasses, namely IgG1, IgG2, IgG3, and IgG4, and IgAl and IgA2.

The heavy chains in IgG, IgA, and IgD antibodies have three constant region domains, which are designated CH1, CH2, and CH3; the heavy chains in IgM and IgE antibodies have four constant region domains, namely CH1, CH2, CH3, and CH4. Thus, the heavy chains have one variable region and three or four constant regions (Harlow et al., Eds., Antibodies: A Laboratory Manual, Chapter 14, Cold Spring Harbor Laboratory, Cold Spring Harbor (1988)).

The human monoclonal anti-idiotype antibodies produced according to the present method are able to specifically recognize and bind the BCRs on the surface of neoplastic lymphocytes of a single patient suffering from a B-cell lymphoproliferative disorder.

In particular, the anti-idiotype antibodies produced according to the present method specifically bind to the variable region of the antibody which is an integrated part of the B-cell receptor (BCR).

Once the anti-idiotype antibodies of the present invention have identified and bound the BCRs on the surface of said neoplastic lymphocytes, they bind to the Fc receptors on the surface of immune cells provided with Fc receptor for immunoglobulins (for example neutrophils, macrophages and Natural Killer cells), thereby allowing destruction of the B lymphocyte by the cell-mediated immune mechanism known as "antibody-dependent cell-mediated cytotoxicity" (ADCC).

The monoclonal anti-idiotype antibodies obtained according to the present method show the noteworthy advantage of being recognized as "self" by the patient they are administered to, since they are totally human as well as highly specific for each single patient; in fact, they are obtained from the blood of the same individual suffering from a B-cell lymphoproliferative disorder whom they are subsequently administered.

As a consequence, the anti-idiotype antibodies obtained according to the method of the present invention show the noteworthy advantage that they do not induce any unwanted immunogenicity effect nor do they induce any neutralizing antibodies once they are administered to the patient.

These advantages guarantee maximal efficiency of anti-idiotype antibodies obtained according to the present invention and, as a consequence, a better treatment of the B-cell lymphoproliferative disorder of the patient compared to that obtained by administering corresponding anti-idiotype antibodies obtained according to the currently known methods, namely murine antibodies.

A further noteworthy advantage of the present method is that it is performed totally in vitro, without involving the use of any laboratory animal.

The advantage of performing the method of the present invention totally in vitro leads to a substantial reduction of the complexity of the production method and its costs, as well as the time required to produce the monoclonal anti-idiotype antibodies.

In fact, the time required for fully performing the method of the present invention is advantageously reduced to a few months (about three months).

Further advantages of the present invention are illustrated and described in detail below.

### DESCRIPTION OF THE INVENTION

The present invention relates to a method for producing human monoclonal anti-idiotype antibodies capable of binding to antibodies produced by patients suffering from a B-cell lymphoproliferative disorder and, subsequently, promoting their elimination.

The first step of the method according to the present invention consists in isolating the IgM produced by B lymphocytes from venous peripheral blood obtained from patients suffering from a B-cell lymphoproliferative disorder.

Herein, the terms "B cells", "B lymphocytes" and "CD 19 lymphocytes" are used interchangeably.

It is possible to isolate the peripheral mononuclear cells (PBMC) present in the peripheral blood of a patient by centrifugation in density gradient, using a synthetic branched high-molecular weight copolymer dissolved in water, for example Ficoll-Paque Plus produced by GE Healthcare or Histopaque-1077 produced by Sigma-Aldrich, both having a density of 1.077 g/ml at 20°C.

Further technical details regarding said polymers are provided in the Example section.

As used herein, the expression "peripheral mononuclear cells" refers to blood cells characterized by the presence of a nucleus, namely lymphocytes and monocytes.

Herein, "peripheral mononuclear cells" and "PBMC" are used interchangeably.

The centrifugation of the blood sample, first diluted and subsequently stratified onto the copolymer, allows the isolation of PBMC in a precise and rapid manner.

In fact, due to their higher density, the erythrocytes, the granulocytes and the dead cells present in the blood sample pass through the layer of synthetic branched high-molecular weight copolymer, while the lymphocytes and the monocytes present in the blood sample accumulate on the plasma gradient limit since they have a lower density.

It is possible to increase the yield of PBMC collection by using specific tubes provided with special barriers (for example, "Leucosep" tubes produced by Greiner, or "Accuspin" tubes produced by Sigma Aldrich) which hold back the red blood cells on the bottom of the tube and which must be previously filled with said synthetic branched high-molecular weight copolymer dissolved in water by centrifugation. It is also possible to use "Accuspin" tubes already loaded with Histopaque copolymer (Accuspin System-Histopaque-1077, produced by Sigma Aldrich).

From said PBMC layer, it is possible to isolate only CD 19 lymphocytes by immunomagnetic separation using specific kits containing magnetic beads linked to a monoclonal anti-CD 19 antibody; advantageously, these kits allow to remove the magnetic beads after separation, in order to allow to perform a subsequent labeling with different beads.

In particular, the immunomagnetic separation is performed in a magnetic field inside which the separation column is directly placed. The latter consists of a plastic support inside which the matrix is located, through which the labeled cells have to pass through, prior hydration with a special buffer solution. The labeling of the cells, being resuspended in the buffer solution, is performed by incubation with magnetic microbeads conjugated to the monoclonal anti-CD 19 antibody, able to simultaneously and selectively recognize the cells and to allow their subsequent binding to the column matrix, at the time of the passage through the magnetic field.

The cells obtained by positive selection, which have remained bound to the matrix, are recovered by removing the column from the magnet and exerting enough pressure, by means of a special plunger, to allow their detachment.

In the present method, small magnetic microbeads (diameter of about 50 nm) having a specific composition (iron oxide and polysaccharides), which makes them totally biodegradable, are used. This avoids the need to detach them after separation and prevents any interference with the viability and function of the B cells which, after separation, can be immediately used for the next steps of the present method.

As an alternative to the isolation of B lymphocytes from the peripheral blood of a patient by centrifugation in density gradient, the method according to the present invention involves the isolation of B lymphocytes directly from whole peripheral blood of the patient. This can be made by using specific kits for whole blood which contain magnetic beads linked to monoclonal anti-CD 19 antibodies.

The direct isolation of B lymphocytes from peripheral blood using said kits advantageously increases the extraction yield and significantly decreases the total time of B lymphocytes isolation compared to said procedure of extraction and isolation of B lymphocytes by centrifugation on synthetic branched high-molecular weight copolymer and subsequent isolation with magnetic beads.

In fact, advantageously, the direct isolation of B lymphocytes from peripheral blood of the patient does not need any preparation of the sample (for example, centrifugation in density gradient).

In patients suffering from B-cell non-Hodgkin lymphomas o B-cell chronic lymphocytic leukemia, up to 90% of CD 19 lymphocytes thereby obtained are tumor cells.

Subsequently, B lymphocytes thereby obtained are cultured in a standard medium and incubated for about 20-30 days.

It is known that, typically, B lymphocytes spontaneously release the IgM antibodies, which are on their cell membrane, into the culture medium (Vitetta ES, Uhr JW. Cell surface immunoglobulin. V. Release from murine splenic lymphocytes. J Exp Med. 1972 Oct 1; 136(4):676-96).

In fact, the membrane receptors BCRs of B lymphocytes, which comprise a class M (or D) immunoglobulin, undergo a protein "turnover", during which the immunoglobulin is released into the extracellular space and becomes a monomeric IgM (Vitetta et al., 1972).

When the incubation time was finished, the B cells were eliminated by centrifugation and the supernatant, containing the IgM antibodies released by the lymphocytes, was purified by affinity chromatography (Fast Protein Liquid Chromatography (FPLC).

The thus-obtained IgMs are the target idiotype of the human monoclonal anti-idiotype antibodies obtained according to the method of the present invention.

After being purified, the tumor IgMs were biotinylated using common commercially available kits.

The second step of the method of the present invention consists in the isolation of B lymphocytes from healthy individuals.

Each B lymphocyte expresses on its membrane a specific antibody which will be produced in large amounts following antigenic stimulation and transformation into plasma cell. The B lymphocytes of each individual collectively constitute the so-called "repertoire" and produce the pool of antibodies in the blood.

By putting together a large number of B lymphocytes from different individuals, it is therefore possible to obtain a much wider repertoire. The wider is the initial repertoire of B lymphocytes, the higher is the probability that a BCR having affinity for the idiotype of the neoplastic BCR is present in the pool.

The aim of this second step is therefore to obtain a very wide pool of CD 19 lymphocytes from different (healthy) individuals which, in the next step of the present method, will be contacted with the neoplastic IgMs obtained as above (namely the IgMs released into the culture medium by the tumor lymphocytes due to the turnover of the membrane BCR of said lymphocytes).

To reach this aim, buffy coats were used.

By the term "buffy coat", as used herein, is meant a waste product of the processing of blood bags collected in transfusion centers from volunteer donors. In particular, from a blood bag of about 450 ml, red blood cells, platelets and plasma (which will be infused in patients) are separated by cell separators, while the remainder in the bag, comprising the PBMC mostly consisting of B lymphocytes, is concentrated in a volume of about 50 ml and is generally eliminated.

Advantageously, the present method thus allows to take advantage of a waste product of blood donations, i.e. the buffy coat.

Being characterized by a high PBCM concentration, the buffy coats used in the present invention should be adequately diluted in order to be subjected to centrifugation in density gradient on one of the above-mentioned special copolymers.

In fact, as already illustrated above in the first step of the present method, also in this step the above-mentioned centrifugation step is followed by B lymphocyte labeling with magnetic beads and subsequent cell isolation by exposure to a magnet.

At the end of this step, a variegated pool of B lymphocytes from healthy individuals was obtained.

The third step of the method according to the present invention consists in contacting the biotinylated tumor IgMs obtained from the first above-mentioned step with the wide repertoire of B lymphocytes obtained from the above-mentioned second step.

The tumor IgMs (idiotype) will bind to a limited number of BCRs (anti-idiotype) of B lymphocytes and the isolation of the latter will allow the subsequent production of the anti-idiotype antibody (Step 4).

In particular, once the B lymphocytes obtained from buffy coat according to the above-mentioned procedure are thawed, they are incubated with non-specific human IgMs to saturate non-specific binding sites and to bind the Fc receptor (FcR) for IgMs physiologically present on the surface of B lymphocytes. The cells are incubated for 30 minutes, then washed and resuspended in complete medium to reactivate cell metabolism and to allow endocytosis of the FcµR/IgM complex.

The cells are again centrifuged and resuspended in labeling buffer at +4°C to halt cell metabolism in order to stop the turnover of membrane proteins.

Subsequently, the biotinylated tumor IgMs were added to the thus-obtained B lymphocytes and an incubation at 4°C was performed for about 10-20 minutes; in this step, the biotinylated tumor IgMs bind to the anti-idiotype B-cell receptors (BCRs) present on the surface of some B lymphocytes with whom they have a higher binding affinity.

After removing the non-bound biotinylated IgMs, the thus-obtained suspension of B lymphocytes and biotinylated IgMs is then contacted with a suspension of streptavidin-conjugated magnetic beads; the strong binding between the biotin conjugated to the tumor IgMs and the streptavidin conjugated with said beads allows the formation of a complex consisting of magnetic beads-tumor IgMs-B lymphocytes.

The passage of said suspension through special magnetic columns allows the isolation of a limited number of B lymphocytes with a BCR having affinity for neoplastic IgMs.

By using special elution buffers, the B lymphocytes complexed with the magnetic beads and linked to tumor IgMs were released; they are the B lymphocytes able to produce human anti-tumor IgM antibodies, i.e. the human anti-idiotype antibodies of the present invention.

The fourth step of the method according to the present invention consists in the production of cell lines able to express the anti-idiotype antibodies of the present invention.

First of all, the anti-idiotype lymphocytes obtained as above are diluted in 96-well plates so as to isolate a single cell for each well. After cell lysis by rapid freezing-thawing, a reverse-transcription reaction (RT-PCR) on plate is prepared in order to obtain, from the RNA of each cell in each plate well, the respective cDNA to use for the PCR amplification of the variable regions of the heavy chain (VH) and the light chain (VL) of the immunoglobulins expressed by the single lymphocyte.

Said RT-PCR reaction may be prepared using 1µM of each primer specific for the constant regions of the heavy chain (IgM) or the light chains kappa and lambda or 2 µM of Random Hexamers and 50U of SuperScriptTM Reverse Transcriptase.

Subsequently, the variable regions VH and VL of the cDNA obtained as above were amplified by two nested-PCR reactions.

After the second PCR, amplified products were obtained, which were specific for the variable regions VH and VL expressed by the single lymphocyte sequenced to obtain the specific sequences of the antibodies to clone.

In a subsequent step, the amplified products specific for the variable regions of the heavy chain and the light chain of each anti-idiotype antibody, which were obtained from a single B lymphocyte, were sequenced and inserted in a eukaryotic bicistronic expression vector containing the respective constant regions Igµ, Igk and Ig 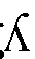 .

As used herein, the term "bicistronic" refers to a system wherein a ribosome can synthesize a polypeptide from an mRNA in a eukaryotic cell, thereby allowing the synthesis of different proteins from a single mRNA.

The vector thus comprises the whole gene of the heavy chain (HC) and the light chain (LC) of each anti-idiotype antibody; both genes were controlled upstream by the CMV (Human cytomegalovirus immediate-early) promoter and had downstream the sequence of transcription termination BGH poly(A). Moreover, the coding sequences HC and LC had at the N-terminus a signal peptide for the export of the protein into the extracellular space. The bicistronic vector also comprises the gene for resistance to neomycin useful for the selection of the cellular clones which have randomly integrated the gene expression cassette inside their genome.

Said vector may optionally comprise additional regulatory sequences such as enhancers, promoters, ribosome binding sites (RBS) and termination signals at the 5' and 3' of the untranslated gene regions, in order to ensure that the gene coding sequence is correctly transcribed and translated.

In order to create stable lines, CHO-K1 cells (Chinese Hamster Ovary cells) were used, which are a cell type typically used for producing recombinant proteins and antibodies. Before transfection, the bicistronic vector was linearized to reduce the probability of integration and causing the disruption of a gene or of other elements necessary for transcription. After transfection, the cells were subjected to selective pressure by neomycin to promote the selection of clones in which the antibody gene was stably integrated. Then, a last dilution in 96-well plates was performed for the purpose of selecting, by ELISA assay, 3 clones having the highest antibody production. The 3 cellular clones were expanded and stored, creating a cell bank for the production of the anti-idiotype antibodies of interest. The stable cell lines were characterized by firstly verifying the presence of the expression cassette inside the genome by DNA extraction and PCR amplification with specific primers of the genes HC and LC and subsequent sequencing.

### Treatment of B-cell lymphoproliferative disorders

The antibodies of the present invention may be used for the treatment of B-cell lymphoproliferative disorders of a subject in need thereof.

As used herein, the expression "B-cell lymphoproliferative disorder" refers to diseases caused by an uncontrolled accumulation of lymphocytes and comprise, but are not limited to, follicular lymphoma (FL), chronic lymphocytic leukemia (CLL), B-cell non-Hodgkin lymphoma, acute lymphoblastic leukemia, hairy cell leukemia (HCL), B-cell lymphomas including diffuse large B-cell lymphoma (DLBCL), multiple myeloma, Waldenström's macroglobulinemia (WM), post-transplant lymphoproliferative disorder (PTLD) and Epstein-Barr virus-associated B-cell lymphoproliferative disorders.

The terms "subject" and "patient" are herein used interchangeably with reference to a human subject.

In an embodiment of the present invention, the anti-idiotype antibodies of the present invention are administered in the presence of a pharmaceutically acceptable vehicle.

"Pharmaceutically acceptable vehicle", as used herein, refers to a diluent, excipient or carrier with which at least one antibody of the present invention can be administered.

The antibodies of the present invention may be administered by any available and efficient delivery system, comprising administration by parenteral route, in particular by subcutaneous, intravenous or intramuscular injection, or infusion techniques.

The pharmaceutical preparations or compositions comprising the antibodies of the present invention for use in the treatment of B-cell lymphoproliferative disorders may be produced using conventional pharmaceutical techniques, as described in the various pharmacopoeia or handbooks of the art such as, for example, "Remington's Pharmaceutical Sciences Handbook", Mack Publishing, New York, 18th Ed., 1990.

Further features and advantages of the present invention will be clear from the following examples provided by way of non-limiting illustration.

### EXAMPLE

### Step 1 - Isolation of IgMs produced by B lymphocytes of a patient suffering from Chronic Lymphocytic Leukemia

Upon authorization by the Ethics Committee of the general hospital ("Policlinico") of Tor Vergata (Rome), three patients suffering from Chronic Lymphocytic Leukemia, who were under the care of the onco-hematology ward, were recruited. After providing their informed consent, each of the three patients were taken a blood draw of 10 ml venous peripheral blood with tube containing EDTA.

In patients suffering from Chronic Lymphocytic Leukemia, the absolute majority of CD 19 lymphocytes circulating in the blood consists of tumor cells (generally with percentages up to 90%).

Each blood sample was filtered with a 30 micron filter ("Pre-separation filters", code 130-041-407, Miltenyi Biotec) using the kits "StraightFrom whole blood CD 19 microbeads human" and "Whole blood column kit" (Miltenyi Biotec) to eliminate possible aggregates.

50 microL of "StraightFrom whole blood CD 19 microbeads" per ml of whole blood with anticoagulant (corresponding to 500 microL) were then added.

After thorough mixing, the sample was incubated in the refrigerator at +4°C for 15 minutes. The cells were then washed adding 20 ml Separation buffer produced by mixing a bottle of "AutoMACS rinsing solution" (code 130-091-222, Miltenyi Biotec) with a bottle of "MACS BSA stock solution" (code 130-091-376, Miltenyi Biotec) and centrifuged at 445 g for 10 minutes at room temperature in a swinging rotor without brake.

Once the supernatant was discarded, the pellet was resuspended in Separation buffer till 1 ml volume was reached.

For each of the three samples, a "Whole blood column kit" (code 130-093-545, Miltenyi Biotec) column was prepared, inserted in the magnet "MidiMACS Separator" (code 130-042-302, Miltenyi Biotec) and installed on "MACS Multistand" (code 130-042-303, Miltenyi Biotec). Each column was washed with 3 ml Separation buffer, then the cell suspension was added. Two consecutive washes with 2 ml Separation buffer were performed and the flow-through was discarded.

Once the column was taken off from the magnetic separator, the CD 19 lymphocytes labeled with the magnetic beads were collected by adding 4 ml "Whole blood column elution buffer" contained in the corresponding above-mentioned kit by Miltenyi Biotec. The buffer was vigorously pushed using the plunger specially provided in the kit and the cells were collected in a tube centrifuged at 500 g for 5 minutes at room temperature.

The supernatant was discarded and the pellet was resuspended to have 10 million tumor CD 19 lymphocytes in 10 ml RPMI 1640 + 10% FBS and 5% glutamine. The so-resuspended cells were put in a 25 ml flask and placed in an incubator at 37°C and 5% CO₂ for 20 days.

After the incubation time was finished, the flask content was centrifuged at 500 g for 5 minutes at room temperature. The supernatant of the cell cultures was concentrated by Amicon Ultra 10K Centrifugal Filter Devices (Millipore) centrifuging at 4000 x g for 15 minutes at a final volume of 5 ml.

The IgMs were purified from the previously concentrated supernatants by Fast Protein Liquid Chromatography, by using chromatographic columns specific for the purification of human IgMs ("LigaTrap Human IgM purification").

In particular, the chromatographic column was first equilibrated with an equilibration buffer (10 mg/ml adipic acid; 800 mM sodium chloride; pH 5.8). For each purification, 5 mL of sample were loaded in the column. The elution occurred in pH gradient (from 4.5 to 4.0) in 0.1M acetate buffer; 1 ml fractions were collected. When the chromatography was finished, the eluted fractions were neutralized by adding a neutralization buffer (1.0 M Tris-base, pH 8.5).

After purification, IgM-positive fractions were identified by Western Blot. In particular, the proteins were separated by electrophoretic run on polyacrylamide (12%) gel under denaturing conditions (SDS-PAGE) and then transferred onto PVDF membrane. After the transfer, the membrane was first incubated with the monoclonal anti-human IgM antibody "Goat Anti-Human IgM mu chain" (ab9169, Abcam) for 1 hour at room temperature and subsequently incubated with the peroxidase-conjugated secondary antibody Donkey Anti-Goat IgG (ab97110, Abcam) for IgM detection. IgM quantification was performed by spectrophotometric techniques (UV-vis spectroscopy).

The purified IgMs were concentrated using Amicon Ultra 3K Centrifugal Filter Devices (Millipore) to reach optimal concentration and volume for biotin conjugation.

IgM biotinylation was performed using the commercial kit Biotin (type B) Fast Conjugation Kit (Abcam) according to the procedure disclosed in the kit: adding 1 µl of Biotin Modifier reagent every 10 µl of the antibody to conjugate, shaking and transferring the mixture into the Biotin Conjugation Mix; incubating for 15 minutes at room temperature; adding 1 µl of Biotin Quencher every 10 µl of antibody and shaking. The obtained conjugate does not require purification. The used kit is specific for the conjugation of biotin to antibodies to be used in assays involving the use of streptavidin immobilized on surfaces (for example magnetic beads).

### Step 2: Isolation of B lymphocytes from healthy individuals.

50 ml buffy coat were diluted 1:5 in PBS (200 ml PBS for 50 ml buffy coat, to a final volume of 250 ml).

Seven 50 ml Falcon tubes were each filled with 15 ml Ficoll Paque PLUS (GE Healthcare) at room temperature. In each tube, 35 ml of pre-diluted buffy coat were then gently stratified.

In particular, Ficoll-Paque Plus is an aqueous solution of density 1.077 + 0.001 g / ml containing 5.7 g "Ficoll 400" and 9 g sodium diatrizoate with 0.0231 g calcium disodium ethylenediamintetraacetic acid in every 100 ml.

"Ficoll 400" is a synthetic high-molecular weight (about 400,000 Da) polymer of sucrose and epichlorohydrin which is readily soluble in water; the molecules of "Ficoll 400" are highly branched, approximately spherical and compactly coiled with a Stokes' radius of about 10 nm.

The Falcon tubes were then centrifuged without brake at 400 g for 35 minutes at 20°C. The PBMC ring, well visible at the interface between the plasma and Ficoll Paque PLUS, was gently sucked with a Pasteur pipette and transferred into a fresh Falcon tube.

The volume was brought to 50 ml with PBS and three washes were performed by centrifugation at 300 g for 10 minutes at 20°C. When the third wash was finished, the supernatant was discarded, the PBMC pellets were collected into a single 50 ml Falcon and resuspended in a final volume of 50 ml PBS.

The cells were counted and then "REAlease CD 19 microbead kit" (catalog nr. 130-117-034 Miltenyi Biotec) was applied according to the relative protocol.

Starting from 170 million PBMCs, the cells were filtered with a 30 micron filter ("pre-separation filters", catalog nr. 130-041-407, Miltenyi Biotec) in order to obtain a solution of single cells and to eliminate cellular aggregates which could obstruct the column.

The suspension was centrifuged at 300 g for 10 minutes at room temperature, then the pellet was resuspended in 680 microL Separation buffer ("AutoMACS rinsing solution", catalog nr. 130-091-222, Miltenyi Biotec mixed with "MACS BSA stock solution", catalog r. 130-091-376, Miltenyi Biotec) at room temperature.

170 microL "REAlease CD 19-biotin" were added and, after homogeneously mixing, the samples were incubated for 5 minutes at room temperature.

850 microL "REAlease anti-biotin microbeads" (CD 19) were added, thoroughly mixing and incubating for 5 minutes.

The subsequent magnetic separation of B lymphocytes labeled with magnetic beads was performed as follows.

An LS column (catalog nr. 130-042-401, Miltenyi Biotec) is positioned onto the magnet "MidiMACS Separator" (code 130-042-302, Miltenyi Biotec) and installed on "MACS Multistand" (code 130-042-303 Miltenyi Biotec). Each column was washed with 3 ml Separation buffer, and then said suspension of B lymphocytes was added.

Three consecutive washes with 3 ml Separation buffer were performed and the flow-through was discarded.

Once the column was taken off from the magnetic separator, the labeled CD 19 lymphocytes were collected by adding 5 ml "REAlease bead release buffer" contained in the kit. The buffer was vigorously pushed using the plunger specially provided in the kit and the cells were collected, incubated for 10 minutes at room temperature to detach the magnetic beads, and counted.

After centrifugation at 500 g for 5 minutes at room temperature, the cells were resuspended in freezing medium (90% FBS + 10% DMSO) at 5 million/ml concentration. Each ml of suspension (containing 5 million cells) was transferred into a single cryovial. The cryovials were inserted into a special device (Mr. Frosty, Nalgene) which had to be placed for 24 hours at -80°C and they were then stored in liquid nitrogen.

### STEP 3 - Production of anti-idiotype B lymphocytes.

20 million CD 19 lymphocytes obtained from two buffy coats from two different volunteers according to the procedure illustrated in Step 2 were thawed.

Thawing occurred by partially thawing the cryovials in thermal bath at 37°C, rapidly adding 5 ml FBS, centrifuging at 500g for 5 minutes at room temperature and resuspending the pellet in 10 ml complete culture medium (RPMI 1640 with 10% FBS) in a 25 ml flask.

The flask was placed in an incubator at 37°C and 5% CO2 overnight.

The day after, a dilution of the biotinylated IgMs, obtained as described in Step 2, at 10⁻³ ng/ml concentration in Labeling buffer (consisting of 1X PBS and 4 mM EDTA at pH 7.2) was prepared. The dilution of idiotypic IgMs was transferred in a 15 ml Falcon tube and stored in ice until use.

Subsequently, the B cells contained in the flask were washed three times with 10 ml Labeling buffer to remove biotin and resuspended in 4 ml Labeling buffer at +4°C, thereby obtaining a concentration of 5 million B lymphocytes / ml.

100 microg human monoclonal IgMs (Raybiotech Inc, catalog 229-20085) were added to the B lymphocytes to neutralize the FcµR receptors physiologically present on the surface of B lymphocytes, incubating the cells in ice for 30 minutes.

The B lymphocytes were then centrifuged, resuspended in 4 ml complete medium RPMI-1640 and incubated at 37°C for 10 minutes to allow endocytosis of the IgM/FcµR complex. The cell suspension was again centrifuged, and the cells were resuspended in 3 ml Labeling buffer at +4°C to halt cell metabolism.

1 ml of the solution of biotinylated idiotype IgMs was then added to the B lymphocyte suspension and was incubated in the refrigerator at +4°C for 15 minutes.

After centrifugation of the thus-obtained cell suspension at 500g for 5 minutes, the pellet was resuspended in 2 ml Labeling buffer.

After the cells had been counted, the cell suspension was centrifuged again at 500g for 5 minutes and the pellet was resuspended in 180 microL Labeling buffer.

20 microL Streptavidin microbeads (code 130-048-101, Miltenyi Biotec), provided in the relative kit, were added to the thus-obtained cell suspension and, after mixing, the suspension was placed in the refrigerator at +4°C for 15 minutes.

Subsequently, after addition of 4 ml Labeling buffer, the cell suspension was centrifuged at 500g for 5 minutes and the pellet was resuspended in 500 microL Separation buffer (obtained by adding 0.5% BSA to the Labeling buffer).

An MS column from Miltenyi Biotec (code 130-042-201) was prepared, inserted in the magnet "MiniMACS Separator" from Miltenyi Biotec (code 130-042-102) and installed on Miltenyi "MACS Multistand" (code 130-042-303). The column was washed with 500 microL Separation buffer, then the cell suspension was added. Three consecutive washes with 500 microL Separation buffer were performed and the flow-through was discarded. Once the column was taken off from the magnetic separator, the lymphocytes labeled with the magnetic beads were collected by adding 1 ml Separation buffer. The buffer was vigorously pushed using the plunger specially provided in the kit, the cells were collected in a Petri and observed under the microscope to confirm the presence of cells.

Said cells were immediately subjected to the procedure described in Step 4.

### STEP 4 - Production of cell lines

Three 96-well plates containing 5 µl/well of 1X SSIV RT Buffer (Invitrogen) were prepared; 5 µl/well of a cell suspension of lymphocytes, obtained according to Step 3, at a density of 200 cells/ml (limiting dilution) were then dispensed. The plates were then immediately frozen at -80°C and subsequently thawed to produce cell lysis.

The RT reaction was then prepared by using the SuperScript^{™} IV First-Strand Synthesis System kit (Invitrogen) in a total volume of 20 µl/well using 1X SSIV RT Buffer, 0.5mM of each dNTP, 5mM DTT, 0.25% Igepal CA-630 (Sigma), 20U RNase inhibitor, 0.1µM of each primer specific for the constant regions of the heavy chain (IgM) (SEQ ID No 1).

The primers used correspond to SEQ ID No 1, SEQ ID No 16 and SEQ ID No 17. The RT-PCR reaction is obtained by incubating the plates at 23°C for 10 min, 50°C for 60 min and 85°C for 10 min.

The plates were then placed at -20°C for cDNA conservation (up to 3 months).

The cDNA was thawed at room temperature and the first PCR (nested-PCR) was performed using a pool of sense primers specific for the respective leader sequences of VH, Vk or V 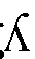 (corresponding to SEQ ID No 2, 4, 5, 6, and 7 for VH; SEQ ID No 18, 19, 20, 21 for Vk region; SEQ ID No 26, 27, 28 for V 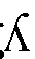 region) each at a concentration of 10µM and a single antisense primer at a concentration of 10 µM specific for the respective constant regions CH (µ), Ck or C 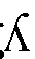 (corresponding to SEQ ID No 8, 32 and 34). The PCR reaction was prepared using the mixture and the DNA polymerase contained in the 2X PCR Master Mix Kit (Diatheva srl) adding the above-described primers at a final concentration of 10µM. PCR conditions were the following: after a 10-minute activation of Taq polymerase at 95°C, 45 cycles consisting of 30 sec at 95°C, 30 sec at 55°C and 45 sec at 72°C followed by a final extension at 72°C for 7 minutes, were carried out.

The second PCR (nested PCR) was prepared from 2 µl of the amplified products of the first PCR by using internal primers specific for Framework 1 regions) of the respective regions VH, Vk or V 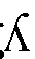 (corresponding to SEQ ID No 9, 10, 11, 12, 13 and 14 for VH; SEQ ID No 22, 23, 24 and 25 for Vk; SEQ ID No 29, 30 and 31 for V 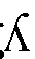 ) and for the respective constant regions (corresponding to SEQ ID No 15, 33 and 35, respectively).

The PCR reaction was prepared using the mixture and the DNA polymerase contained in the 2X PCR Master Mix Kit (Diatheva srl) adding the above-described primers at a final concentration of 10µM. PCR conditions are the following: after a 10-minute activation of Taq polymerase at 95°C, 45 cycles consisting of 30 sec at 95°C, 30 sec at 55°C and 45 sec at 72°C followed by a final extension at 72°C for 7 minutes, were carried out.

When the second nested-PCR was finished, amplified products were obtained, which were specific for the variable regions VH and VL expressed by the single lymphocyte sequenced to obtain the specific sequences of the antibodies to clone.

Table 1 below describes the primers used in RT and in the first and second PCR.

**Table 1: Primers used in the first nested-PCR (1), in the second PCR (2) and in RT-PCR (RT) and respective characteristics of length, melting temperature (TM) and guanine-cytosine percentage (%GC).**

| *NAME* | *PCR* | *Sequence* | *Length* | *TM* | %*GC* |
|---|---|---|---|---|---|
| Cµ CH1 Reverse | RT | SEQ 1: GGGAATTCTCACAGGAGACGA | 21 | 60 | 52.38 |
| VHL-1 Forward | 1 | SEQ 2: TCACCATGGACTGCACCTGGA | 21 | 64 | |
| VHL-2 Forward | 1 | SEQ 3: CCATGGACACACTTTGCTCCAC | 21 | 61 | |
| VHL-3 Forward | 1 | SEQ 4: TCACCATGGAGTTTGGGCTGAGC | 23 | 69.7 | 56 |
| VHL-4 Forward | 1 | SEQ 5: AGAACATGAAACACCTGTGGTTCTT | 25 | 62.8 | 40 |
| VHL-5 Forward | 1 | SEQ 6: ATGGGGTCAACCGCCATCCT | 20 | 67 | 60 |
| VHL-6 Forward | 1 | SEQ 7: ACAATGTCTGTCTCCTTCCTCAT | 23 | 58.7 | 4.3 |
| CµII CH1 Reverse (nested) | 1 | SEQ 8: CAGGAGACGAGGGGGAAAAG | 20 | 62.69 | 60 |
| VH-1 Forward | 2 | SEQ 9: CAGGTGCAGCTGGTGCAGTC | 20 | 62.7 | 65 |
| VH-2 Forward | 2 | SEQ 10: CAGATCACCTTGAAGGAGTC | 20 | 53 | 50 |
| VH-3 Forward | 2 | SEQ 11: GAGGTGCAGCTGGTGGAGTC | 20 | 61.7 | 65 |
| VH-4 Forward | 2 | SEQ 12: CAGGTGCAGCTGCAGGAGTC | | 61.3 | |
| VH-5 Forward | 2 | SEQ 13: GAGGTGCAGCTGGTGCAGTC | | 62.8 | 65 |
| VH-6 Forward | 2 | SEQ 14: CAGGTACAGCTGCAGCAGTC | 20 | 58 | 60 |
| CµIII Reverse | 2 | SEQ 15: AGGTCTAGAGAAAAGGGTTGGGGCGGATGC | | 69 | |

| *NAME* | *PCR* | *Sequence* | *Length* | *TM* | %*GC* |
|---|---|---|---|---|---|
| CκI Reverse | RT | SEQ 16: AACAGAGGCAGTTCCAGA | | 49.87 | 50 |
| CλI Reverse | RT | SEQ 17: TGTGGCCTTGTTGGCTTG | | 58 | 50 |
| VκL-1 Forward | 1 | SEQ 18: GCTCAGCTCCTGGGGCTCCTG | 21 | 69.7 | 71.4 |
| VκL-2 Forward | 1 | SEQ 19: CTGGGGCTGCTAATGCTCTGG | 21 | 66.2 | 61.9 |
| VκL-3 Forward | 1 | SEQ 20: TTCCTCCTGCTACTCTGGCTC | 21 | 60.41 | 57.14 |
| VκL-4 Forward | 1 | SEQ 21: CAGACCCAGGTCTTCATTTCT | 21 | 57.45 | 47.6 |
| Vk-1 Forward | 2 | SEQ 22: CGACATCCAGATGACCCAGT | | 57 | |
| Vk-2 Forward | 2 | SEQ 23: CGATATTGTGATGACCCAG | | | |
| Vk-3 Forward | 2 | SEq24: CGAAATTGTGTTGACGCAGTCT | | 52 | |
| Vk-4 Forward | 2 | SEQ 25: CGACATCGTGATGACCCAGT | | 58 | |
| VλL-1 Forward | 1 | SEQ 26: CCTCTCCTCCTCACCCTCCT | 20 | 61 | 65 |
| VλL-2 Forward | 1 | SEQ 27: CTCCTCACTCAGGGCACA | 18 | 55.4 | 61 |
| VλL-3 Forward | 1 | SEQ 28: ATGGCCTGGATCCCTCTCC | | 62 | |
| Vλ-1 Forward | 2 | SEQ 29: TATTAGATCTCCAGTCTGTGCTGACTCAGC | | | |
| Vλ-2 Forward | 2 | SEQ 30: TATTAGATCTCCAGTCTGCCCTGACTCAGC | | | |
| Vλ-3 Forward | 2 | SEQ 31: CACCAGATCTCTCCTATGAGCTGACTCAGC | | | |
| CκII Reverse | 1 | SEQ 32: TTTCAACTGCTCATCAGATGGCGG | | | |
| CκIII Reverse | 2 | SEQ 33: AAGATGAAGACAGATGGTGC | | 52 | |
| CλII Reverse | 1 | SEQ 34: AGCTCCTCAGAGGAGGGCGG | 20 | 67 | 70 |
| CλIII Reverse | 2 | SEQ 35: CATTCCATGGGGGAACAGAGTGACCG | | 73 | |

Subsequently, the amplified products obtained of the variable regions of the heavy chain and of the light chain of each anti-idiotype antibody, which were obtained from a single B lymphocyte, were inserted in a eukaryotic bicistronic expression vector pCDNA 3.1. provided by ThermoFisher Scientific, according to the following protocol.

The bicistronic vector was linearized and introduced in CHO-K1 cells. Once transfection was finished, the cells were incubated in the presence of neomycin for 2 weeks. The cells that survived neomycin selection were diluted to obtain one cell per well in 96-well plates.

The 3 clones having higher antibody production were selected by evaluating antibody concentration in the culture medium. Subsequently, these clones were expanded and stored. The stable cell lines were characterized by firstly verifying the presence of the expression cassette inside the genome by DNA extraction and PCR amplification with specific primers of the genes HC and LC and subsequent sequencing.

Finally, cell cultures from each of the three lines were established according to techniques known to the skilled experts in the art. The monoclonal antibodies were purified from the culture medium by Fast Protein Liquid Chromatography and antibody affinity to the antigen was measured by Biacore.

## Claims

1. A method for producing human monoclonal anti-idiotype antibodies against the variable region of the antibody comprised in the B-cell receptor (BCR) of a patient suffering from a B-cell lymphoproliferative disorder, said method comprising the following steps:
a) purifying IgM antibodies produced by B cells obtained from a blood sample from said patient suffering from a B-cell lymphoproliferative disorder;
b) purifying B cells from blood samples obtained from one or more healthy individuals;
c) contacting said IgM antibodies obtained from step a) with said B cells obtained from step b), thus forming IgM antibody-B cell complexes;
d) purifying and dissociating said complexes formed in step c), thus obtaining B cells producing said human monoclonal anti-idiotype antibodies.

2. The method according to claim 1, wherein said step a) of purifying IgM antibodies comprises the following steps:
i) centrifuging in density gradient a blood sample from a patient suffering from a B-cell lymphoproliferative disorder, thus obtaining and isolating peripheral mononuclear cells (PBMC);
ii) contacting said peripheral mononuclear cells (PBMC) with monoclonal anti-B-cell antibodies linked to magnetic beads and performing an immunomagnetic separation, thus obtaining purified B cells;
iii) culturing the purified B cells obtained from step ii) ;
iv) purifying the IgM antibodies produced by said B cells in culture.

3. The method according to claim 1, wherein said step a) of purifying IgM antibodies comprises the following steps:
i) contacting a blood sample from a patient suffering from a B-cell lymphoproliferative disorder with monoclonal anti-B-cell antibodies linked to magnetic beads and performing an immunomagnetic separation, thus obtaining purified B cells;
ii) culturing the purified B cells obtained from step i) ;
iii) purifying the IgM antibodies produced by said B cells in culture.

4. The method according to any one of the preceding claims, wherein said step b) of purifying B cells comprises the following steps:
i) centrifuging in density gradient a blood sample from a healthy individual, thus obtaining and isolating peripheral mononuclear cells (PBMC);
ii) contacting said peripheral mononuclear cells (PBMC) with monoclonal anti-B-cell antibodies linked to magnetic beads and performing an immunomagnetic separation, thus obtaining purified B cells.

5. The method according to any one of claims 1-3, wherein said step b) comprises contacting a blood sample from a healthy individual with monoclonal anti-B-cell antibodies linked to magnetic beads and performing an immunomagnetic separation, thus obtaining purified B cells.

6. The method according to any one of the preceding claims, wherein said centrifugation in density gradient is performed in an aqueous solution having a density of 1.077 g/ml at 20°C and comprising a polymer of polysucrose and sodium diatrizoate having a molecular weight between 350.000 and 450.000 Da.

7. The method according to any one of claims 4 to 6, wherein said blood sample is a "buffy coat" of one or more healthy individuals.

8. The method according to any one of the preceding claims, wherein said step c) of forming IgM antibody-B cell complexes comprises the following steps:
i) biotinylating the IgM antibodies obtained from step a);
ii) contacting the B cells obtained from step b) with human polyclonal IgMs under conditions such as to saturate the Fc receptors of said B cells;
iii) contacting the biotinylated IgM antibodies obtained from step i) with the B cells obtained from step ii), thus obtaining B-cell-biotinylated IgM complexes;
iv) contacting said B cell-biotinylated IgM complexes with magnetic beads associated to streptavidin, avidin or neutravidin, thus obtaining B cell-IgM-magnetic bead complexes;
v) isolating said complexes obtained from step iv) by magnetic separation;
vi) isolating said IgM antibody-B cell complexes by dissociating the complexes obtained from step v) from the magnetic beads.

9. The method according to any one of the preceding claims, wherein said B-cell lymphoproliferative disorder is selected from the group comprising follicular lymphoma (FL), chronic lymphocytic leukemia (CLL), B-cell non-Hodgkin lymphoma, acute lymphoblastic leukemia, hairy cell leukemia (HCL), B-cell lymphomas including diffuse large B-cell lymphoma (DLBCL), multiple myeloma, Waldenström's macroglobulinemia (WM), post-transplant lymphoproliferative disorder (PTLD) and Epstein-Barr virus-associated B-cell lymphoproliferative disorders.

10. The method according to claim 9, wherein said B-cell lymphoproliferative disorder is chronic lymphocytic leukemia (CLL) or B-cell non-Hodgkin lymphoma.

11. A human monoclonal anti-idiotype antibody obtained by the method according to any one of claims 1-10.
